# EUROPEAN PATENT APPLICATION

(11) **EP 2 083 086 A1**
(43) Date of publication of application: **29.07.2009**
(21) Application number: 07807859.9
(22) Date of filing: 26.09.2007
(51) Int. Cl.: C12Q 1/42, C12Q 1/32, G01N 21/78

(54) **METHOD FOR FORMATION OF REAGENT LAYER IN ANALYSIS APPARATUS, METHOD FOR MANUFACTURE OF ANALYSIS APPARATUS, AND ANALYSIS APPARATUS**

(30) Priority: 26.09.2006 JP 2006259911
(71) Applicant: Arkray, Inc., Kyoto-shi, Kyoto 601-8045 (JP)
(72) Inventor: SHINOZAKI, Kotaro, Kyoto-shi, Kyoto 601-8045 (JP)
(74) Representative: HOFFMANN EITLE
(86) International application number: PCT/JP2007/068590
(87) International publication number: WO 2008/050566

(57) **Abstract**

The present invention relates to a method for forming a reagent layer in an analytical tool including a first step of holding a reagent-containing liquid onto a substrate, and a second step of drying the reagent-containing liquid. A liquid containing a tetraborate as a drying accelerator is used as the reagent-containing liquid. A content of a tetraborate in the reagent-containing liquid is, for example 1 g or more based on 100 mL of the reagent-containing liquid. The tetraborate is, for example, tetrasodium borate or tetrapotassium borate.

## Description

### TECHNICAL FIELD

The present invention relates to an analytical tool having a reagent layer, and a technique for forming a reagent layer.

### BACKGROUND ART

As a method for analyzing a sample, there is, for example, a method in which a reaction liquid when a sample and a reagent are caused to react with each other is analyzed in an optical technique or an electrochemical technique. When a sample is analyzed by the method, an analytical tool for supplying a reaction field is used. In this case, the analytical tool is used in the state that the tool is fitted to an analytical instrument for analyzing a reaction liquid, and the tool is made to have a reagent layer for being caused to react with a sample.

The reagent layer is usually formed by causing a reagent-containing liquid to adhere, in a dot form, onto a substrate, and then drying the reagent-containing liquid. The composition of the reagent-containing liquid is decided in accordance with a component which is to be a measurement target, a sample-analyzing technique to be used, or the like. When the measurement target is, for example, a biogenic component such as glucose, the composition is prepared to contain a redox enzyme. When a sample is analyzed in an optical technique, the composition is prepared to contain a color-developing agent.

In the meantime, the drying of the reagent-containing liquid is generally attained by natural drying, drying under a low-humidity condition (see, for example, Patent Document 1), or drying using hot air, infrared rays or the like (see, for example, Patent Documents 2 and 3).

However, according to the natural drying or drying under a low-humidity condition, at the time when the reagent-containing liquid contains a large amount of a component high in hygroscopicity or moisturizing property (for example, N-methyl-D-glucamine or 3- (N-morpholino) propanesulfonic acid), a period necessary for drying the reagent-containing liquid becomes long. It is therefore feared that when the reagent-containing liquid is dried, the reagent component therein deteriorates.

Contrary to this method, according to the drying method using hot air or infrared rays, a short time is sufficient for drying the reagent-containing liquid. However, the temperature of the reagent is raised by the hot air or infrared rays. This matter causes a deterioration of a reagent component weak against high temperature that may be contained in the reagent-containing liquid. For example, when the reagent-containing liquid contains a color-developing agent, the color-developing agent is colored. When the reagent-containing liquid contains a redox enzyme, the redox enzyme may be inactivated. When such a reagent component is deteriorated, a target component in the sample is not easily measured with a high precision.

In the meantime, the following method is also supposed: a method of adding a reagent stabilizing agent such as sucrose to a reagent-containing liquid to make the amount of a solvent component such as water relatively small, thereby drying the reagent-containing liquid at a relatively low temperature in a short period without using hot air or infrared rays.

When sucrose is added to a reagent-containing liquid, the drying rate is certainly improved. However, it cannot be mentioned that the drying promotion effect of sucrose is sufficient when the reagent-containing liquid contains a component high in hygroscopicity or moisturizing property. Therefore, when a reagent-containing liquid includes hygroscopicity or moisturizing property, it becomes necessary to increase the addition amount of sucrose in the reagent-containing liquid. As a result, costs of the reagent-containing liquid rise up. Additionally, when the reagent-containing liquid is dried, a bad effect is produced onto the surface property of the reagent layer, or the like, which is based on the matter that the sucrose content is large. More specifically, when the sucrose content in the reagent-containing liquid is large, irregularities may be generated in the surface of the reagent layer formed after the liquid is dried, or the surface of the reagent layer may be cracked. The irregularities or cracks in the reagent layer surface causes the generation of air bubbles when the reagent layer is dissolved, so as to cause not only a measurement error but also unevenness of the solubility of the reagent layer. The unevenness makes the concentration of the reagent uneven to cause another measurement error.

Patent Document 1: JP-A-01-049962
Patent Document 2: JP-A-62-278454
Patent Document 3: JP-A-07-110324

### DISCLOSURE OF THE INVENTION

### PROBLEMS TO BE SOLVED BY THE INVENTION

An object of the present invention is to restrain the generation of irregularities or cracks in the surface of a reagent layer without deteriorating the reagent therein, and make it possible to analyze a target component in a sample with a good precision.

### MEANS FOR SOLVING THE PROBLEMS

According to a first aspect of the present invention, provided is a method for forming a reagent layer in an analytical tool, including a first step of holding a reagent-containing liquid onto a substrate, and a second step of drying the reagent-containing liquid, wherein as the reagent-containing liquid, there is used a liquid containing a tetraborate as a drying accelerator.

According to a second aspect of the present invention, provided is a method for producing an analytical tool having a reagent layer for being caused to react with a sample, including a first step of holding a reagent-containing liquid on a substrate, and a second step of drying the reagent-containing liquid, wherein as the reagent-containing liquid, there is used a liquid containing a tetraborate as a drying accelerator.

The content of the tetraborate in the reagent-containing liquid is for example 1 g or more, and preferably from 1 to 10 g based on 100 mL of the reagent-containing liquid.

It is preferable to use tetrasodium borate or tetrapotassium borate as the tetraborate, and among them, tetrasodium borate is most preferably used.

The reagent-containing liquid may further contain sucrose. The content of sucrose in the reagent-containing liquid is for example from 5 to 15 g based on 100 mL of the reagent-containing liquid.

The reagent-containing liquid contains, for example, at least one of an enzyme and a color-developing agent.

The reagent-containing liquid is held in an amount of, for example, 10 to 100 nL on the substrate in the first step.

The reagent-containing liquid is dried, for example, in an environment having a relative humidity of 10% or less, and preferably dried at room temperature in the second step.

The reagent layer is a layer for analyzing, for example, alkali phosphatase (ALP), glucose or lactate dehydrogenate (LDH), and is preferably formed as a single layer or as a solid layer that is dissolved by a sample.

The analytical tool to be produced in the second aspect of the present invention is a tool further having, for example, a reaction tank for causing the sample and the reagent to react with each other. In this case, the reagent layer is provided in the reaction tank. The analytical tool is a tool used to analyze the sample in an optical technique, for example.

According to a third aspect of the present invention, provided is an analytical tool having a reagent layer for causing a sample and a reagent to react with each other, wherein the reagent layer contains a tetraborate.

The content of the tetraborate in the reagent layer is for example from 3 to 15% by weight.

It is preferable to use tetrasodium borate or tetrapotassium borate as the tetraborate, and among them, tetrasodium borate is most preferably used.

In the analytical tool of the present invention, the reagent layer may further contain sucrose. The content of sucrose in the reagent layer is for example from 5 to 30% by weight.

The reagent layer is formed, for example, as a single layer or as a solid layer that is dissolved by the sample.

The analytical tool according to the present invention may be a tool further having a reaction tank for causing the sample and the reagent to react with each other. In this case, the reagent layer is provided in the reaction tank.

The analytical tool according to the present invention is a tool for analyzing the sample in an optical technique, for example.

### BRIEF DESCRIPTION OF THE DRAWINGS

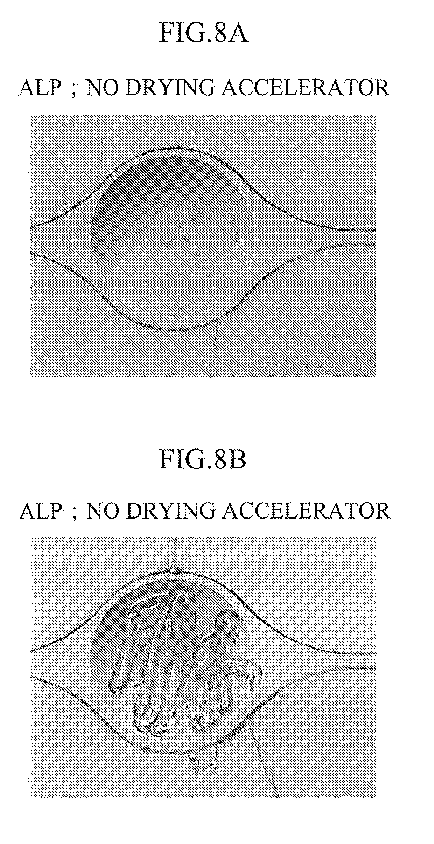

[FIG. 1]

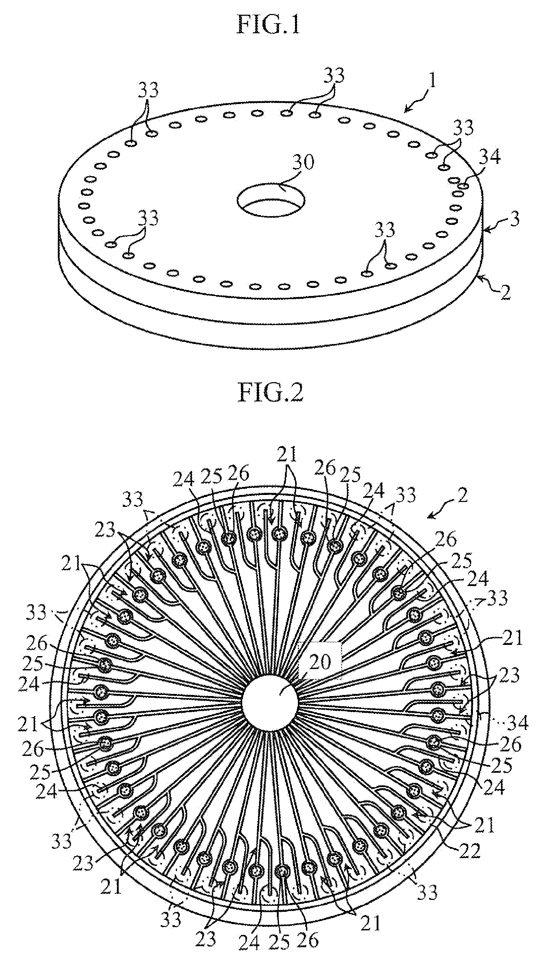

   FIG. 1 is a general perspective view illustrating an example of a micro-device which is to be a production target of the present invention.
[FIG. 2]
   FIG. 2 is a plan view of a substrate in the micro-device illustrated in FIG. 1.
[FIG. 3]

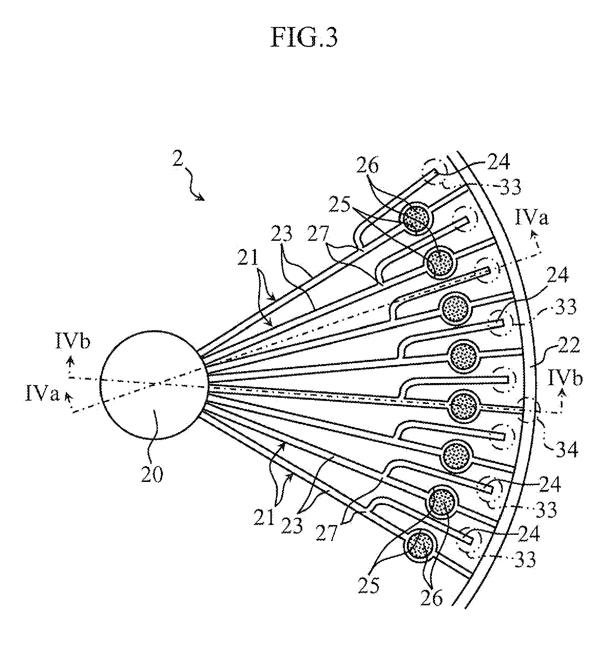

   FIG. 3 is a partial plan view illustrating an enlarged main portion of the substrate.
[FIGs. 4]

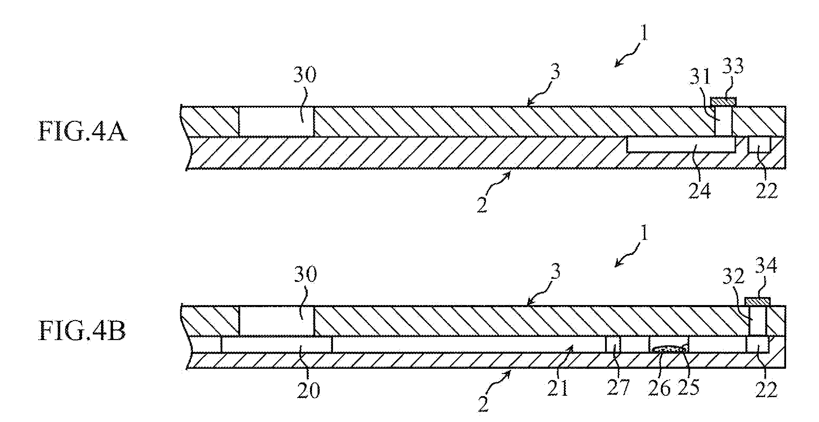

   FIG. 4A is a section view corresponding to the cross section taken along line IVa-IVa of FIG. 3 in the micro-device in FIG. 1, and FIG. 4B is a sectional view corresponding to the cross section taken along line IVb-IVb of FIG. 3 in the micro-device in FIG. 1.
[FIG. 5]

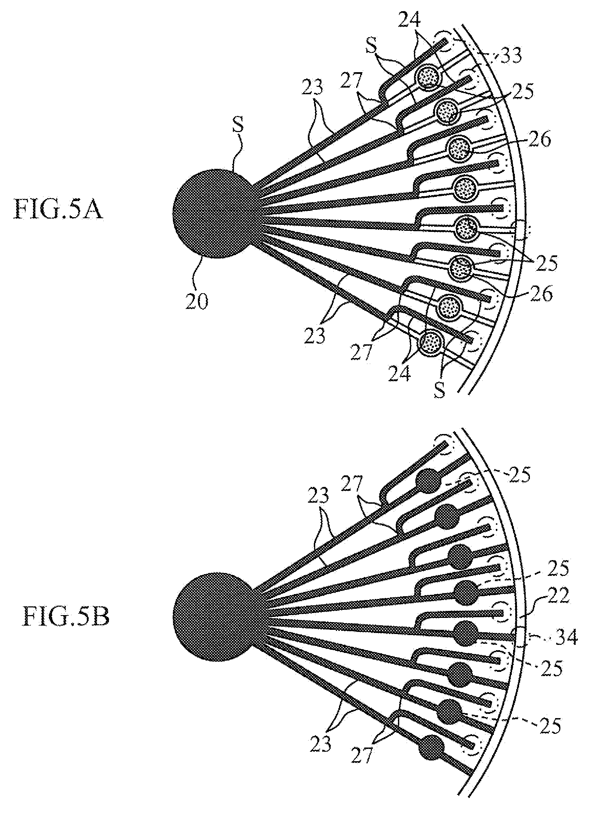

   FIG. 5 is a partial plan view of a substrate corresponding to PIG. 3 in order to describe a state that a sample is transferred in the micro-device illustrated in FIG. 1.
[FIGs. 6]

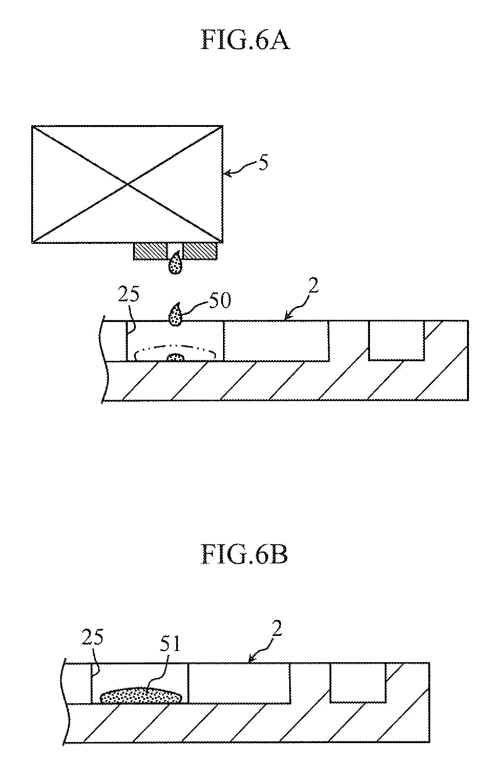

   FIGs. 6A and 6B are each a sectional view in order to describe the step of forming reagent layers on the substrate.
[FIGs. 7]

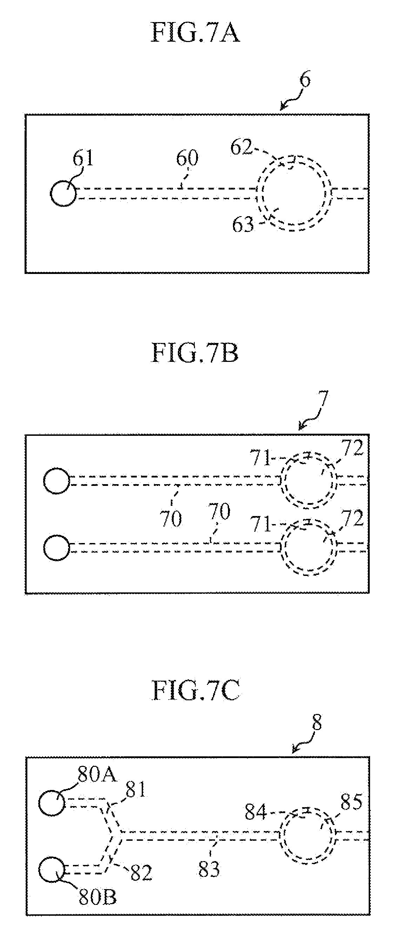

   FIGs. 7A to 7C are each a plan view in order to describe another example of an analytical tool which is to be a production target of the present invention.
[FIGs. 8]
   PIG. 8A is an optically microscopic photograph showing an ALP-measuring reagent layer formed without adding, to a reagent-containing liquid, any tetraborate as a drying accelerator nor sucrose as a reagent stabilizing agent, and FIG. 8B is an optically microscopic photograph showing a state that the reagent layers in FIG. 8A were rubbed with a needle.
[FIGs. 9]

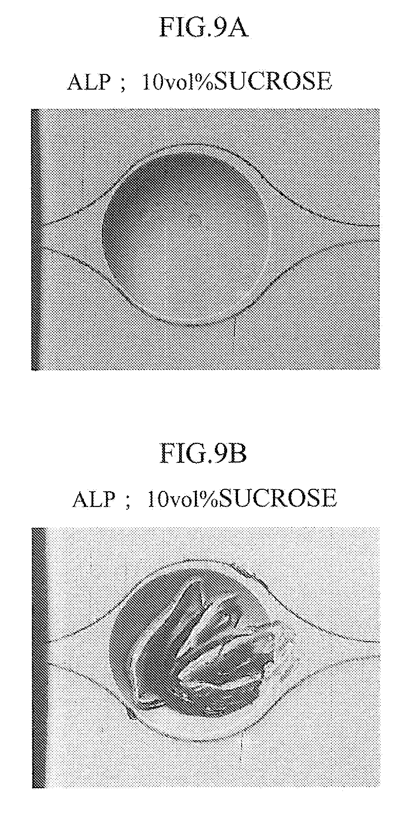

   FIG. 9A is an optically microscopic photograph showing an ALP-measuring reagent layer formed by adding, to the reagent-containing liquid, 10% by volume of sucrose as a reagent stabilizing agent, and FIG. 9B is an optically microscopic photograph showing a state that the reagent layers in FIG. 9A were rubbed with a needle.
[FIGs. 10]

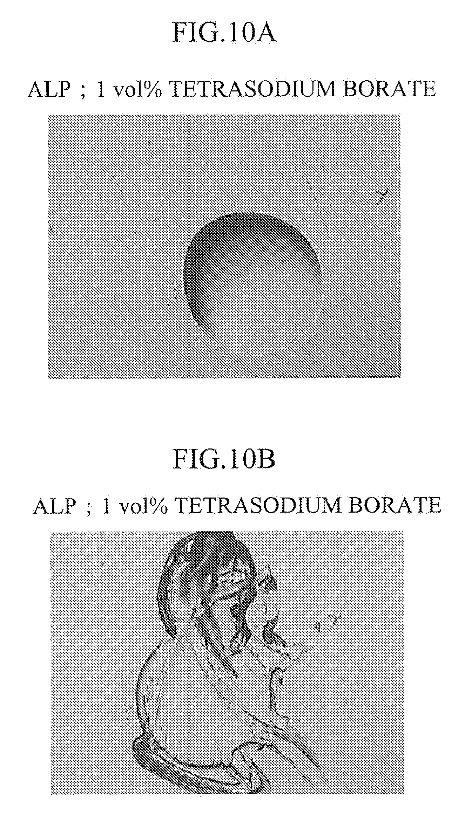

   FIG. 10A is an optically microscopic photograph showing an ALP-measuring reagent layer formed by adding, to the reagent-containing liquid, 1 % by volume of tetrasodium borate as a drying accelerator, and FIG. 10B is an optically microscopic photograph showing a state that the reagent layers in FIG. 10A were rubbed with a needle.
[FIGs. 11]

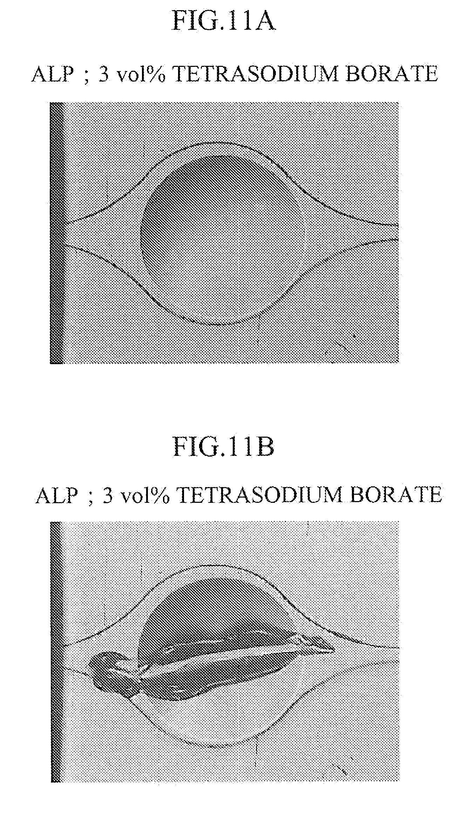

   FIG. 11A is an optically microscopic photograph showing an ALP-measuring reagent layer formed by adding, to the reagent-containing liquid, 3% by volume of tetrasodium borate as a drying accelerator, and FIG. 11B is an optically microscopic photograph showing a state that the reagent layers in FIG. 11A were rubbed with a needle.
[FIGs. 12]

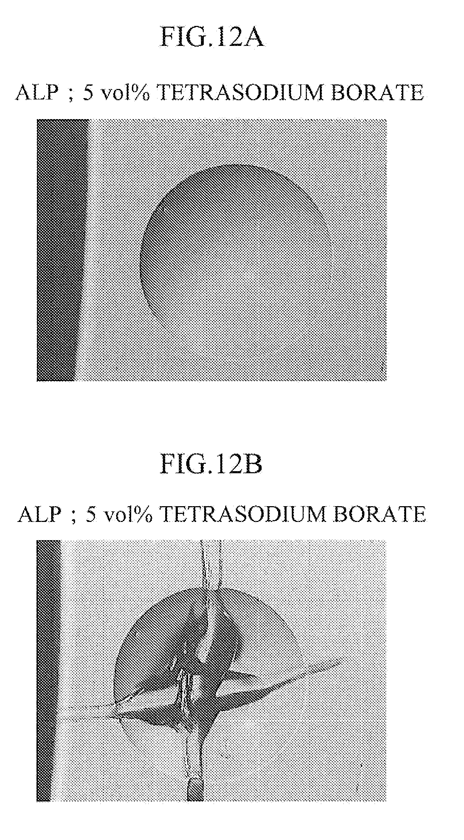

   FIG. 12A is an optically microscopic photograph showing an ALP-measuring reagent layer formed by adding, to the reagent-containing liquid, 5% by volume of tetrasodium borate as a drying accelerator, and FIG. 12B is an optically microscopic photograph showing a state that the reagent layers in FIG. 12A were rubbed with a needle.
[FIGs. 13]

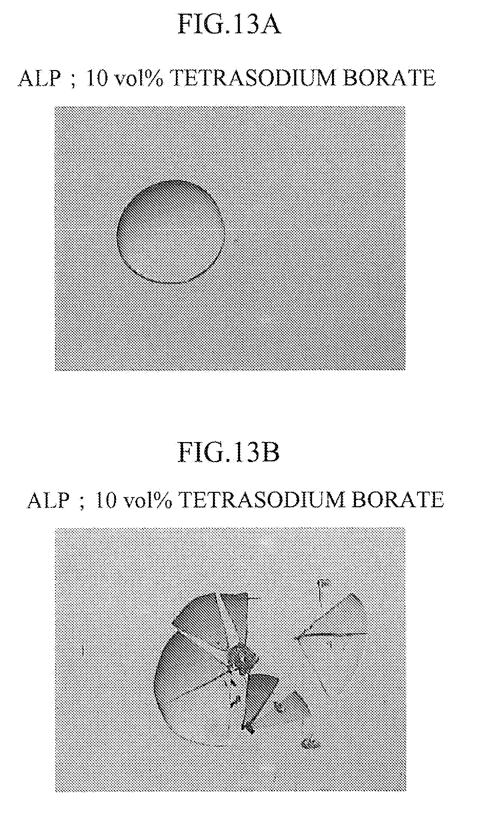

   FIG. 13A is an optically microscopic photograph showing an ALP-measuring reagent layer formed by adding, to the reagent-containing liquid, 10% by volume of tetrasodium borate as a drying accelerator, and FIG. 13B is an optically microscopic photograph showing a state that the reagent layers in FIG. 13A were rubbed with a needle.
[FIGs. 14]

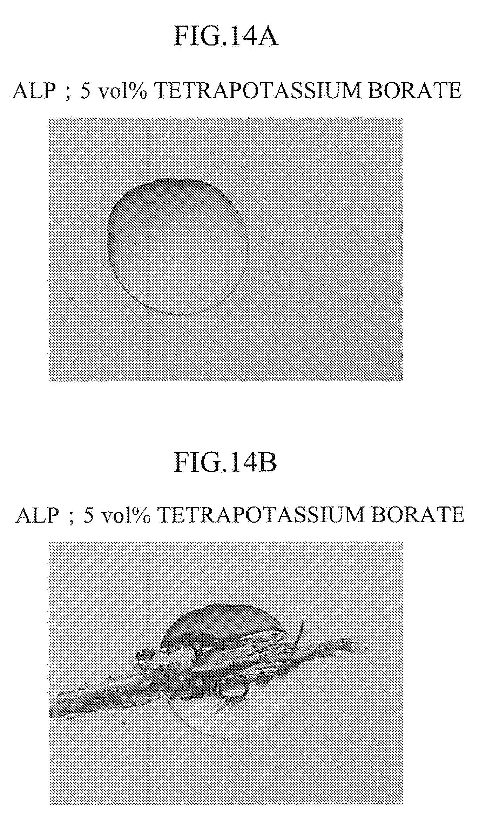

   FIG. 14A is an optically microscopic photograph showing an ALP-measuring reagent layer formed by adding, to the reagent-containing liquid, 5% by volume of tetrapotassium borate as a drying accelerator, and FIG. 14B is an optically microscopic photograph showing a state that the reagent layers in FIG. 14A were rubbed with a needle.
[FIGs. 15]

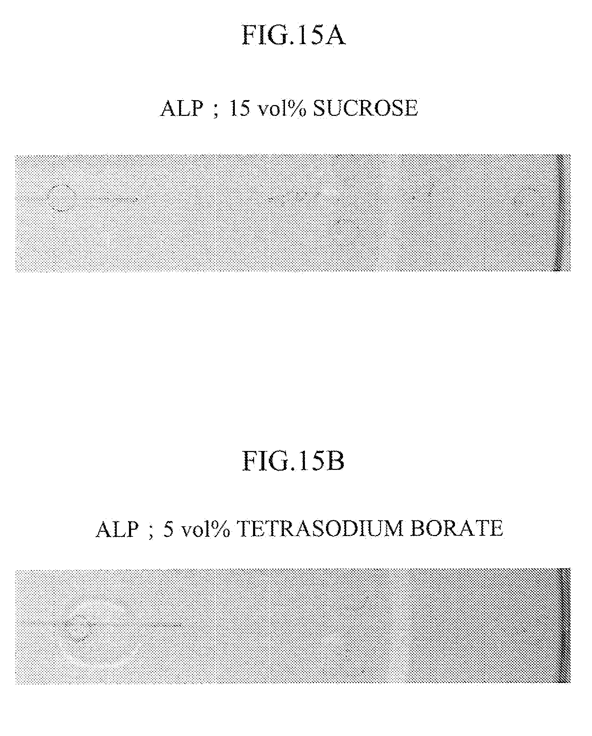

   FIG. 15A is a microscopic photograph showing a state that reagent layers was dissolved in a micro-device, the reagent layer being formed by adding, to a reagent-containing liquid, sucrose as a reagent stabilizing agent, and FIG. 15B is a microscopic photograph showing a state that reagent layers were dissolved in a micro-device, the reagent layers being formed by adding, to the reagent-containing liquid, tetrasodium borate as a drying accelerator.
[FIGs. 16]

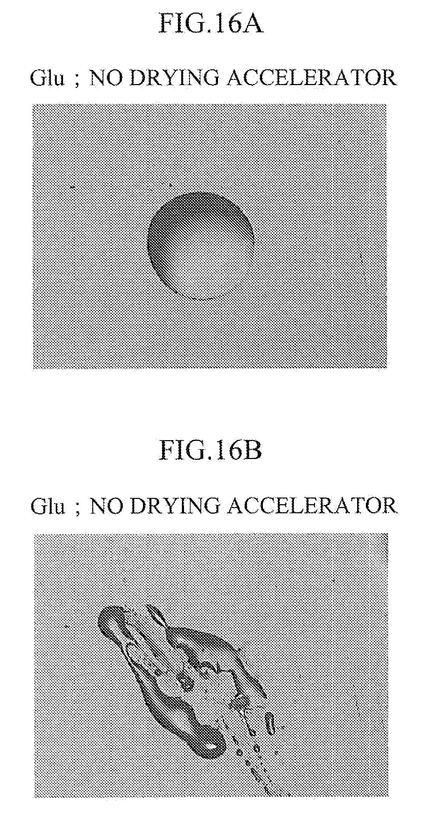

   FIG. 16A is an optically microscopic photograph showing a glucose-measuring reagent layer formed without adding, to a reagent-containing liquid, any tetraborate as a drying accelerator nor sucrose as a reagent stabilizing agent, and FIG. 16B is an optically microscopic photograph showing a state that the reagent layers in PIG. 6A were rubbed with a needle.
[FIGs. 17]

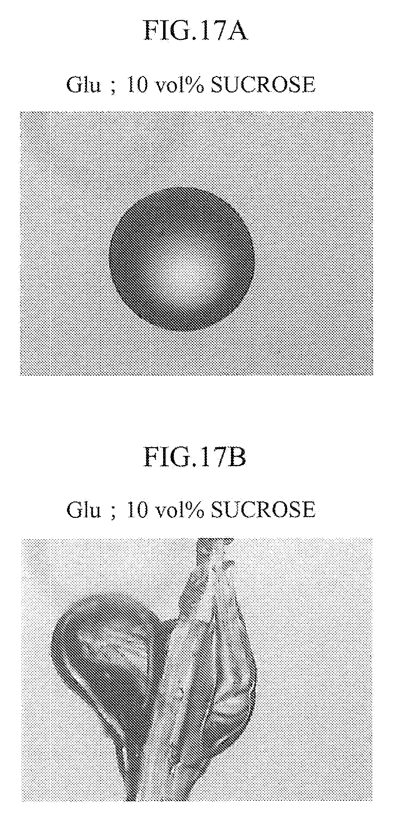

   FIG. 17A is an optically microscopic photograph showing a glucose-measuring reagent layer formed by adding, to the reagent-containing liquid, 10% by volume of sucrose as a reagent stabilizing agent, and FIG. 17B is an optically microscopic photograph showing a state that the reagent layers in FIG. 17A were rubbed with a needle.
[FIGs. 18]

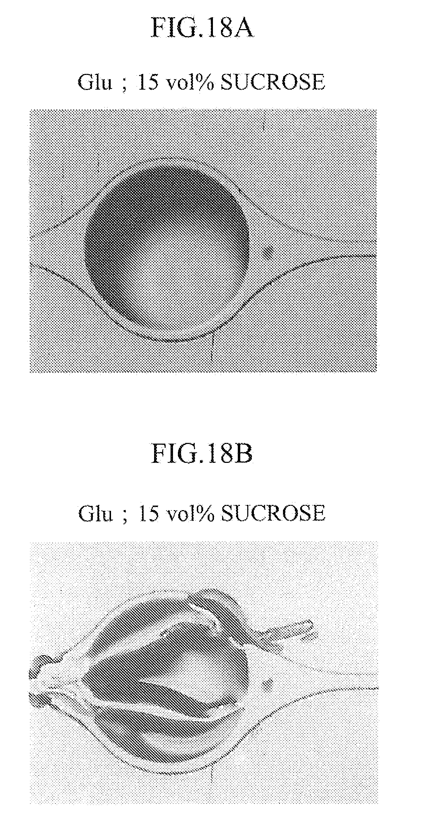

   FIG. 18A is an optically microscopic photograph showing a glucose-measuring reagent layer formed by adding, to the reagent-containing liquid, 15% by volume of sucrose as a reagent stabilizing agent, and FIG. 18B is an optically microscopic photograph showing a state that the reagent layers in FIG. 18A were rubbed with a needle.
[FIGs. 19]

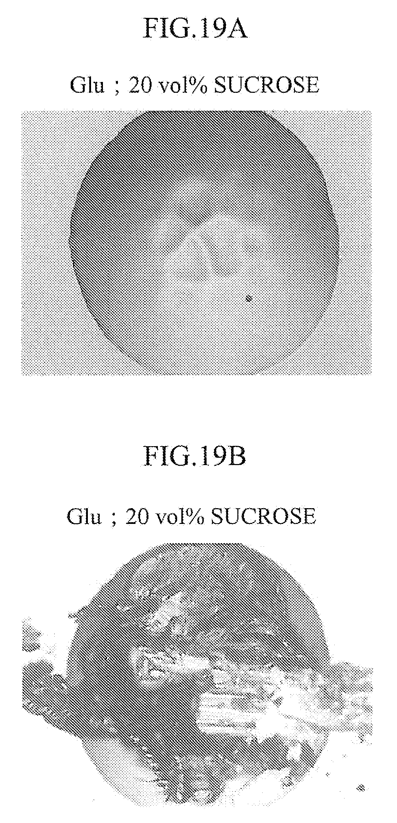

   FIG. 19A is an optically microscopic photograph showing a glucose-measuring reagent layer formed by adding, to the reagent-containing liquid, 20% by volume of sucrose as a reagent stabilizing agent, and FIG. 19B is an optically microscopic photograph showing a state that the reagent layers in FIG. 18A were rubbed with a needle.
[FIGs. 20]

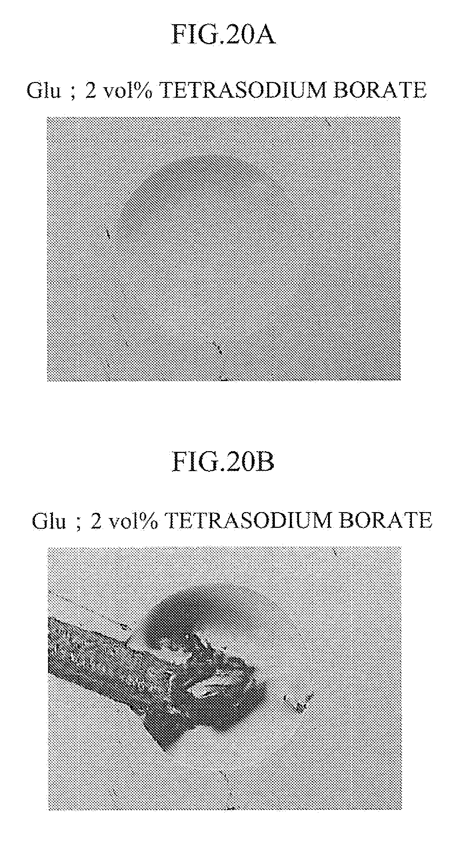

   FIG. 20A is an optically microscopic photograph showing a glucose-measuring reagent layer formed by adding, to the reagent-containing liquid, 2% by volume of tetrasodium borate as a drying accelerator, and FIG. 20B is an optically microscopic photograph showing a state that the reagent layers in FIG. 20A were rubbed with a needle.
[FIGs. 21]

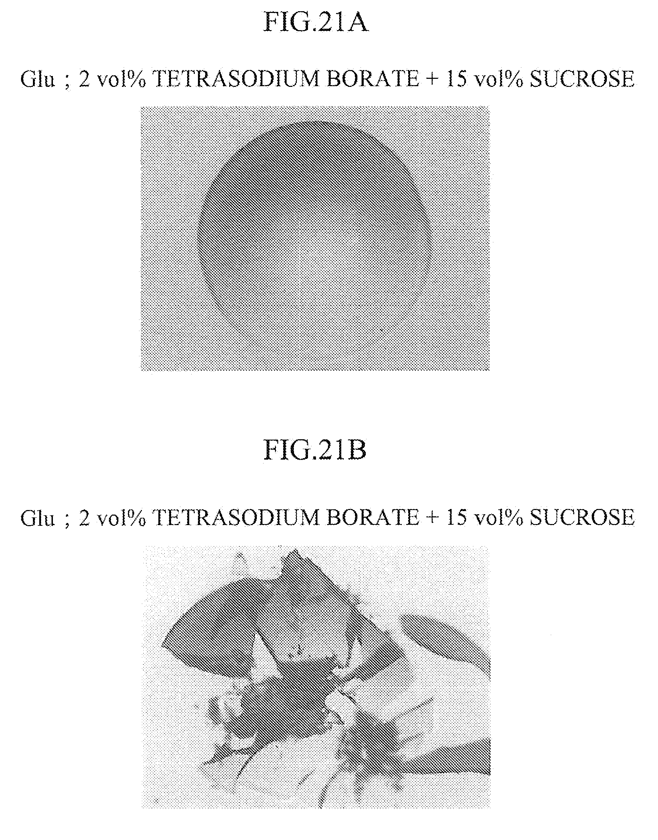

   FIG. 21A is an optically microscopic photograph showing a glucose-measuring reagent layer formed by adding, to the reagent-containing liquid, 2% by volume of tetrasodium borate as a drying accelerator and 15% by volume of sucrose, and FIG. 21B is an optically microscopic photograph showing a state that the reagent layers in FIG. 21A were rubbed with a needle.
[FIGs. 22]

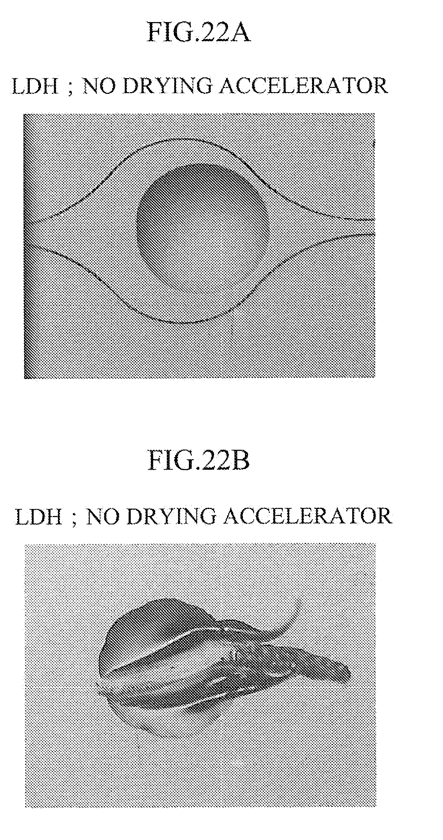

   FIG. 22A is an optically microscopic photograph showing an LDH-measuring reagent layer formed without adding, to a reagent-containing liquid, any tetraborate as a drying accelerator nor sucrose as a reagent stabilizing agent, and FIG. 22B is an optically microscopic photograph showing a state that the reagent layers in FIG. 22A were rubbed with a needle.
[FIGs. 23]

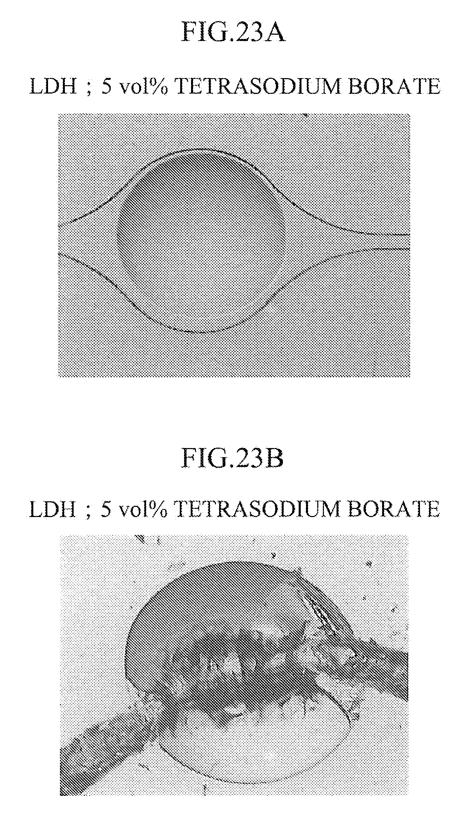

   FIG. 23A is an optically microscopic photograph showing an LDH-measuring reagent layer formed by adding, to the reagent-containing liquid, 5% by volume of tetrasodium borate as a drying accelerator, and FIG. 23B is an optically microscopic photograph showing a state that the reagent layers in FIG. 23A were rubbed with a needle.

### REFERENCE NUMERALS

1,6,7 and 8 micro-devices (analytical tools)
2 base plate (substrate)
25, 62, 71, and 84 reaction cells (reaction tanks)
26 reagent layers
50 and 51 reagent-containing liquids

### Best Mode for Carrying Out the Invention

With reference to the drawings, the present invention will be described hereinafter.

First, a micro-device will be described which is an example of an analytical tool that is to be a production target in the present invention.

A micro-device 1 illustrated in FIG. 1 is a device used when a sample is analyzed in an optical technique and used in the state that the device is fitted to an analytic instrument (not illustrated). This micro-device 1 is constructed as a disposable device, and has a substrate 2 and a cover 3.

As illustrated in FIGs. 2 to 4, the substrate 2 is formed into a disc form as a whole, and is equipped with a liquid-receiving section 20, plural channels 21, and a common channel 22.

The liquid-receiving section 20 is a section for holding a sample to be introduced into each of the channels 21, and is formed as a columnar concave in the center of the substrate 2.

The channels 21 are members for transferring a sample by capillarity, and are provided in a radial form as a whole. Each of the channels 21 has a main channel 23 and a branched channel 24.

The main channel 23 has a reaction cell 25, and is connected to the common channel 22. This main channel 23 is constructed in such a manner that capillarity acts by discharging a gas in the common channel 22 outside. This reaction cell 25 provides a field where a sample and a reagent are caused to react with each other, and is further a section functioning as a light-measuring area. A reagent layer 26 is provided inside the cell.

The reagent layers 26 are formed suitable for analyzing, for example, alkali phosphatase (ALP), glucose (Glu), lactate dehydrogenase (LDH), albumin (Alb), total bilirubin (T-Bil), inorganic phosphorus (IP), urea (UA), urea nitrogen (BUN), aspartic acid aminotransferase (GOT), alanine aminotransferase (GPT), creatine phosphokinase (CPK), amylase (Amy), γ-glutamyltranspeptitase (GGT), creatinine (Cre), total proteins (TP), calcium (Ca), magnesium (Mg), fructosamine (FRA), total cholesterol (T-Cho), high-density lipoprotein cholesterol (HDL-Cho), low-density lipoprotein cholesterol (LDL-Cho), or triglyceride neutral fat (Tg). The reagent layers 26 are each a layer containing a tetraborate as a drying accelerator, and are each formed, for example, as a solid single layer that is dissolved when brought into contact with a sample.

The tetraborate is, for example, tetrasodium borate or tetrapotassium borate. The content by percentage of the tetraborate in the reagent layers 26 is, for example, from 3 to 15% by weight.

The reagent layers 26 are formed to contain a color-developing agent, an electron transfer material, a redox enzyme, a surfactant, a preservative or the like in accordance with a specific component to be analyzed. The reagent layers 26 may be layers wherein sucrose is contained in order to dry the reagent layers 26 easily when the layers 26 are formed. The content by percentage of sucrose in the reagent layers 26 is set into, for example, 5 to 30% by weight.

The branched channels 24 are each a channel for attaining the state that a sample is supplied to the front of the reaction cell 25. The channel is branched from the main channel 21 at a branched portion 27 positioned at a somewhat upstream side of the reaction cell 25. As illustrated in FIG. 4A, this branched channel 24 is connected to one out of discharge ports 31 for branched channel, which will be described later, in the cover 3.

As illustrated in FIG. 2, the common channel 22 is a channel used when gas in the branched channels 24 is discharged, and is formed in a ring form in the circumference of the substrate 2. As illustrated in FIG. 4B, this common channel 22 is connected to discharge ports 32 for common channel, which will be described later, in the cover 3.

As illustrated in FIGs. 1 and 4, the cover 3 is formed in the form of a transparent disc as a whole, and has a sample introduction port 30, the discharge ports 31 for branched channel, and the discharge ports 32 for common channel.

The sample introduction port 30 is a port used when a sample is introduced, and is made as a through hole. The sample introduction port 30 is made in the center of the cover 3 to be positioned just above a liquid-receiving region 20 of the substrate 2.

Each of the discharge ports 31 for branched channel is made as a through hole connected to the corresponding branched channel 24. An upper opening in each of the discharge ports 32 for common port is closed with a sealing member 33.

As illustrated in FIG. 5A, when openings are made in the sealing members 33 in the state that a sample S is held in the liquid receiving region 20, the upstream sides of the branched portions 27 in the main channels 23 and the branched channels 24 are connected to the outside. Capillarity acts onto these portions. For this reason, the sample S in the liquid receiving region 20 is transferred at the upstream sides of the main channels 23, and then introduced to the branched channels 24. As a result, the sample S turns in the state that the sample S is stopped close to the reaction cells 25.

As illustrated in FIGs. 1 and 4, the discharge ports 32 for common channel are ports for discharging gas in the main channels 23, and are made as through holes. The discharge ports 32 for common channel are made in the substrate 2 to be positioned just above predetermined positions of the common channel 21, and upper openings thereof are closed with sealing members 34.

As illustrated in FIG. 5B, when openings are made in the sealing members 34, the downstream sides of the branched portions 27 in the main channels 23 are connected to the outside, and capillarity acts onto these portions. For this reason, the sample S that has reached close to the reaction cells 25 is introduced to the reaction cells 25. As a result, the reagent layers 26 are dissolved by the sample S so that a target component in the sample S in the reaction ceils 25 and the reagent react with each other. A reaction product from the target component in the sample S and the reagent is detected by means of a known light-measuring mechanism. On the basis of the detection result, the target component is analyzed.

The following will describe a method for producing the micro-device 1.

In the production of the micro-device 1, the substrate 2 and the cover 3, which are each in the above-mentioned form, are first formed.

The substrate 2 and the cover 3 are formed by resin-molding using, a transparent resin material, for example, an acrylic resin such as polymethyl methacrylate (PMMA), polydimethylsiloxane (PDMS) or polystyrene (PS). In the resin-molding, the shape of the mold is well designed, thereby forming the liquid receiving region 20, the channels 21 and the common channel 22 about the substrate 2 and the sample introduction port 30, the discharge ports 31 for branched channel, and the discharge ports 32 for common channel at the same time.

Next, the reagent layers 26 are formed in the reaction cells 25 in the substrate 2. The reagent layers 26 are formed through a dot-adhering step of causing a reagent-containing liquid to adhere, into a dot form, onto the reaction cell 25, and a drying step of drying the reagent-containing liquid.

As illustrated in FIG. 6A, the dot-adhesion of the reagent-containing liquid is attained, for example, by repeating dot-adhesion of one or more minute-amount liquid droplets 50 of the reagent-containing liquid through an inkjet head 5. The amount of (any one of) the droplet(s) 50 is set into, for example, about 10 to 300 pL. The number of the droplets for forming any one of the reagent layers 26 is set into, for example, about 10 to 800, and the total dot-adhesion amount for forming any one of the reagent layers 26 is set into, for example, 10 to 100 nL. In the case of attaining the dot-adhesion of a predetermined amount of the reagent-containing liquid onto each of the reaction cells 25 in this way, the reagent-containing liquid 51 is held in a dome form in the reaction cell 26 as illustrated in FIG. 6B.

Of course, it is not necessarily essential that the dot-adhesion for the reagent layers 26 is attained by the repeated dot-adhesion of the minute-amount droplet (s) 50. The dot-adhesion may be attained by performing dot-adhesion of a predetermined amount of the reagent-containing liquid at a time by use of a nozzle or the like.

As the reagent-containing liquid, there is used, for example, a liquid containing a reagent and a tetraborate as a drying accelerator. If necessary, it is allowable to incorporate, thereinto, distilled water, a buffer solution, a preservative, a surfactant or some other additive besides the above components.

The composition of the reagent in the reagent-containing liquid may be appropriately selected in accordance with the kind of the target component to be analyzed. Examples of the reagent include a color-developing agent, an electron transfer material, and a redox enzyme.

The tetraborate is preferably tetrasodium borate or tetrapotassium borate, most preferably tetrasodium borate. The content of the tetraborate in the reagent-containing liquid is set, for example, to 1 g or more, preferably into the range of 1 to 10 g based on 100 mL of the reagent-containing liquid. This is based on the following: if the addition amount of the tetraborate is too small, a sufficient drying promotion effect is not obtained; on the other hand, if the addition amount of the tetraborate is too large, the tetraborate may not be sufficiently dissolved in the reagent-containing liquid.

Sucrose may be added as a reagent stabilizing agent to the reagent-containing liquid to make the drying period short. The addition amount of sucrose in this case is set into such a range that irregularities and cracks are not generated in the surfaces of the reagent layers 26 formed by drying the reagent-containing liquid, and is set, for example, into the range of 5 to 15 g based on 100 mL of the reagent-containing liquid.

In the meantime, the drying step is performed by keeping the substrate 2, on which the reagent-containing liquid 51 is held, still in a predetermined environment. The predetermined environment in the drying step is, for example, an environment wherein the relative humidity is 10% or less and the temperature is room temperature or normal temperature (about 20 to 30°C). The period for drying the reagent-containing liquid is decided in accordance with the composition of the reagent-containing liquid, elements of which include the kind and the amount of the drying accelerator, and is usually set into the range of 2 to 12 hours.

Of course, the relative humidity, the temperature and the drying period in the drying step are not limited to the above-mentioned ranges as far as the reagent is not deteriorated, and may be variously modified.

When the drying of the reagent-containing liquid is completed to form the reagent layers 26, the substrate 2 and the cover 3 are jointed to each other, thereby forming the micro-device 1 illustrated in FIG. 1. The joint between the substrate 2 and the cover 3 may be attained, for example, by use of a hot melt adhesive.

In the present invention, the reagent-containing liquid 51, which contains a tetraborate as a drying accelerator, is used to form the reagent layers 26. The inventors have confirmed that this tetraborate is higher in drying promotion effect than sucrose, which is conventionally used as a reagent stabilizing agent. Therefore, even if the reagent-containing liquid contains therein a component high in hygroscopicity or moisturizing property (such as N-methyl-D-glucamine or 3-(N-morpholino)propanesulfonic acid) in a large amount, irregularities are not generated in the surfaces of the reagent layers 26 formed after the drying or cracks are not generated in the surfaces of the reagent layers 26 so that the surface property can be made stable. As a result, when the sample S is introduced into the reaction cell 25 to dissolve the reagent layers 26, the generation of air bubbles can be restrained. Additionally, the reagent layers 26 can be substantially evenly dissolved to make the concentration of the reagent even in the solution. In this way, the reagent layers 26 formed in the present invention, or in turn an analytical tool formed by the present invention makes it possible to overcome measurement errors.

When the tetraborate as a drying accelerator is incorporated into the reagent-containing liquid 51, the reagent-containing liquid 51 can be dried in a relatively short period even by natural drying or drying under a low-humidity condition. Thus, a deterioration of the reagent component can be restrained in the drying of the reagent-containing liquid 51, so as to restrain a fall in measurement precision which results from a deterioration of the reagent component.

Of curse, the present invention is not limited to the above-mentioned embodiment, and may be variously modified. For example, the above-mentioned embodiment has been described while one out of micro-devices wherein plural channels are provided in a radial form is given as an object to which the present invention is applied. However, the present invention can be applied to a case where an analytical tool in a different form is produced or a case where reagent layers of the analytical tool are formed. Analytical tools to which the present invention is to be applied are, for example, devices illustrated in FIGs. 7A to 7C besides the micro-device 1.

An analytical tool 6 illustrated in FIG. 7A is a tool wherein a single channel 60 is formed. More specifically, the analytical tool 6 is a tool wherein a sample introduced from a sample introduction port 61 is supplied to a reaction cell 62, and then a reagent layer 63 is dissolved by the sample in the reaction cell 62.

An analytical tool 7 illustrated in FIG. 7B is a tool wherein plural channels 70 (the number of which is two in the figure) are provided in parallel to each other. A reaction cell 71 (a reagent layer 72) is set in each of the channels 70. According to this analytical tool 7, about a single sample, plural items can be simultaneously analyzed, or plural samples can be simultaneously analyzed about the same item.

An analytical tool 8 illustrated in FIG. 7C is a tool wherein samples or reagents introduced from sample introduction ports 80A and 80B are individually transferred in individual channels 81 and 82, and then jointed with each other at a common channel 83 to supply the samples in a single reaction cell 84 (a reagent layer 85) set to the common channel 83.

The present invention is not limited to any analytical tool wherein a sample is transferred by capillarity, and may be applied to an analytical tool wherein a sample is transferred by an external driving force such as a pump, or a reagent layer of an analytical tool wherein a sample is analyzed in an optical technique or a reagent layer of an analytical tool wherein a sample is analyzed in an electrochemical technique.

The following will describe the present invention by way of Examples. However, the present invention is not limited to Examples described below.

### Example 1

In the present example, drying promotion effect was examined in a case where at the time of forming reagent layers suitable for measuring alkali phosphatase (ALP), tetrasodium borate or tetrapotassium borate was added to a reagent-containing liquid.

The reagent layers were formed by using a dispenser to cause the reagent-containing liquid to adhere in a dot form onto a substrate made of polystyrene (PS), and then drying the resultant in a night. As the reagent-containing liquid, prepared was each liquid wherein each drying accelerator or reagent stabilizing agent shown in Table 2 described below was added to a basic composition shown in Table 1 described below. The dot-adhesion amount of the reagent-containing liquid was set to 50 nL.

The drying promote effect was investigated by checking, with an optical microscope, the property of the reagent layers after the drying, and the property thereof after the reagent layers after the drying were rubbed with a needle. Optically microscopic photographs of the reagent layers after the drying are shown in FIGs. 8A to 14A, and optically microscopic photographs of the reagent layers after the layers were rubbed with the needle are shown in FIGs. 8B to 14B.

**Table 1**

| Reagent component | Concentration |
|---|---|
| MEG | 1800 mmol/L |
| Nacl | 300 mmol/L |
| MgCl₂·6H₂O | 1.8 mmol/L |
| CHAPSO | 2 vol% |
| Distilled water | |

The pH of the reagent-containing liquid was adjusted to 10.6 with 5 N HC1.
MEG:N-methyl-D-glucamine
CHAPSO:3-[(3-cholamidopropyl)dimethylammonio]-2-hydro xypropanesulfonate

**Table 2**

| Formulation | Drying accelerator/Reagent stabilizing agent | Concentration |
|---|---|---|
| A | Not contained | |
| B | Sucrose | 10 vol% |
| C | Tetrasodium borate | 1 vol% |
| D | Tetrasodium borate | 3 vol% |
| E | Tetrasodium borate | 5 vol% |
| F | Tetrasodium borate | 10 vol% |
| G | Tetrapotassium borate | 5 vol% |

As understood form FIG. 8A, in the formulation A, wherein neither any drying accelerator nor any reagent stabilizing agent was added, separation of the reagent was recognized in the reagent layers. On the other hand, as illustrated in FIG. 8B, it was confirmed that when the reagent layers were rubbed with the needle, the reagent layers contained a large amount of water so that the reagent layers were hardly dried.

As understood from FIG. 9, in the formulation B, wherein sucrose as a reagent stabilizing agent was added to the reagent-containing liquid (except sucrose) in an amount of 10% by volume of the liquid, separation of the reagent in the reagent layers was recognized but the separation of the reagent in the reagent layers was made better than the case where neither any reagent stabilizing agent nor any drying accelerator was added. On the other hand, as illustrated in FIG. 9B, it was confirmed that when the reagent layers were rubbed with the needle, the reagent layers were not completely dried but the drying of the reagent layers was further promoted as compared with the case where no drying accelerator was added.

On the other hand, as illustrated in FIG. 10A, in the formulation C, wherein tetrasodium borate as a drying accelerator was added to the reagent-containing liquid (except tetrasodium borate) in an amount of 1% by volume of the liquid, no separation of the reagent in the reagent layers was recognized, which was different from the case where neither any drying accelerator nor any reagent stabilizing agent was added, or the case where sucrose as a reagent stabilizing agent was added. As illustrated in FIG. 10B, it was confirmed that when the reagent layers were rubbed with the needle, the reagent layers were not completely dried but the drying of the reagent layers was further promoted as compared with the case where neither any drying accelerator nor any reagent stabilizing agent was added.

As illustrated in FIG. 11A, in the formulation D, wherein tetrasodium borate as a drying accelerator was added to the reagent-containing liquid (except tetrasodium borate) in an amount of 3% by volume of the liquid, no separation of the reagent in the reagent layers was recognized and neither irregularities nor cracks were generated. As illustrated in FIG. 11B, it was confirmed that when the reagent layers were rubbed with the needle, the reagent layers were not completely dried but the drying of the reagent layers was promoted equivalently to or largely than the case where sucrose as a reagent stabilizing agent was not added in an amount of 10% by volume. In other words, it was confirmed that when tetrasodium borate was used as a drying accelerator, a small addition amount thereof gave drying promotion effect equal to or larger than that based on sucrose as compared with the case where sucrose as a reagent stabilizing agent was used in order to make the drying period short.

As understood from FIG 12A, in the formulation E, wherein tetrasodium borate as a drying accelerator was added to the reagent-containing liquid (except tetrasodium borate) in an amount of 5% by volume of the liquid, no separation of the reagent in the reagent layers was recognized and neither generation of irregularities nor that of cracks was recognized. As illustrated in FIG. 12B, it was confirmed that when the reagent layers were rubbed with the needle, the drying of the reagent layers was further promoted as compared with the case where Tetrasodium borate was added in an amount of 3% by volume.

As understood from FIG. 13A, in the formulation F, wherein tetrasodium borate was added to the reagent-containing liquid (except tetrasodium borate) in an amount of 10% by volume of the liquid, no separation of the reagent in the reagent layers was recognized. As illustrated in FIG. 13B, it was confirmed that when the reagent layers were rubbed with the needle, the reagent layers were completely dried to such a degree that the reagent layers were cracked.

On the other hand, as understood from FIG. 14A, in the formulation G, wherein tetrapotassium borate as a drying accelerator was added to the reagent-containing liquid (except tetrapotassium borate) in an amount of 5% by volume of the liquid, no separation of the reagent in the reagent layers was recognized. As illustrated in FIG. 14B, it was confirmed that when the reagent layers were rubbed with the needle, the reagent layers were not completely dried but the drying of the reagent layers was still further promoted as compared with the case where no drying accelerator was added.

From the above-mentioned results, it has been understood that when tetrasodium borate is added as a drying accelerator, the drying of the reagent-containing liquid is promoted and further the drying promotion effect is more effectively obtained on the basis of a smaller addition amount than when sucrose as a reagent stabilizing agent, which is to be added to make the drying period short, is added. Additionally, it has been understood that when tetrapotassium borate is used as a drying accelerator, the same results as when tetrasodium borate is used are also obtained.

### Example 2

In the present example, about micro-devices each having reagent layers formed by the method of Example 1, measurement reproducibility was examined.

The micro-devices were made into a form similar to the form as illustrated in FIG. 7A. The reagent layers were formed in the same way as in Example 1. However, the substrates and the covers in the micro-devices were made of polystyrene (PS). As reagent-containing liquids for forming the reagent layers, the following were used: a reagent-containing liquid wherein sucrose was incorporated as a reagent stabilizing agent into the basic composition in Table 1 in an amount of 15% by volume of the composition; and a reagent-containing liquid wherein tetrasodium borate was incorporated as a drying accelerator into the basic composition in an amount of 5% by volume of the composition. The dot-adhesions of the reagent-containing liquids were conduced, using a dispenser. The dot-adhesion amounts were each set to 50 nL.

About each of the case where sucrose was added and the case where tetrasodium borate was added, distilled water was supplied to ten out of the micro-devices. The absorbances of their reaction cells at this time were measured. In this way, the measurement reproducibility was examined. The results of the measured absorbances are shown in Tables 3 and 4 described below.

About micro-devices selected at will from the ten micro-devices, a situation of the generation of air bubbles was checked with a microscope. A microscopic photograph in the case where sucrose was added is shown in FIG. 15A, and that in the case where tetrasodium borate was added is shown in FIG. 15B.

**Table 3**

| Drying accelerator (reagent stabilizing agent) | Absorbance (mAbs/min) | | | | | | | | | |
|---|---|---|---|---|---|---|---|---|---|---|
| Sucrose: 15 vol% | 9.6 | 9.5 | 9.3 | 9.3 | 8.7 | 8.8 | 9.0 | 11.1 | 9.3 | 9.4 |
| Tetrasodium borate: 5 vol% | 10.6 | 10.9 | 11.4 | 10.3 | 10.8 | 10.7 | 10.6 | 10.9 | 10.5 | 10.5 |

**Table 4**

| Drying accelerator (reagent stabilizing agent) | Average value (mAbs/min | Standard deviation | Reproducibility (%) |
|---|---|---|---|
| Sucrose: 15 vol% | 9.4 | 0.7 | 7.1 |
| Tetrasodium borate: 5 vol% | 10.7 | 0.3 | 2.9 |

As understood from Tables 3 and 4, the measured absorbances were more stable and the measurement reproducibility was better when tetrasodium borate was used as a drying accelerator than when sucrose was used as a reagent stabilizing agent in order to make the drying period short.

As illustrated in FIG. 15A, it was confirmed that when sucrose was used as a reagent stabilizing agent, air bubbles were generated in the reaction cells. On the other hand, as illustrated in FIG. 15B, it was not confirmed that when tetrasodium borate was used as a drying accelerator, air bubbles were generated in the reaction cells.

From the above-mentioned result, it appears that: when sucrose is used as a reagent stabilizing agent, the surface property of the reagent layers deteriorates; therefore, when the reagent layers are dissolved, air bubbles are generated so that the measured absorbance is not stabilized so as to deteriorate the reproducibility. On the other hand, it appears that: when tetrasodium borate is used as a drying accelerator, the surface property of the reagent layers is improved; therefore, when the reagent layers are dissolved, no air bubbles are generated so that the measured absorbance is stabilized so as to improve the reproducibility. Thus, when tetrasodium borate is used as a drying accelerator, measurement precision can be improved. It is assumed that such an advantageous effect can be obtained when tetrapotassium borate is used as a drying accelerator as well as when tetrasodium borate is used.

### Example 3

In the present example, drying promotion effect was examined in a case where tetrasodium borate was added when reagent layers suitable for measuring glucose (Glu) were formed.

The reagent layers were formed by causing each reagent-containing liquid to adhere in a dot form onto a substrate made of polystyrene (PS), and then drying the resultant in a night. The reagent-containing liquid was prepared by adding each drying accelerator or each reagent stabilizing agent shown in Table 6 described below to a basic composition shown in Table 5 described below. The dot-adhesion of the reagent-containing liquid was performed, using a dispenser. The dot-adhesion amount thereof was set to 50 nL.

The drying promotion effect was judged by checking the property of the reagent layers after the drying, and the property thereof after the reagent layers after the drying were rubbed with a needle. Optically microscopic photographs of the reagent layers after the drying are shown in FIG. 16A to FIG. 21A, and optically microscopic photographs of the reagent layers after the reagent layers were rubbed with the needle are shown in FIG. 16B to FIG. 21B.

**Table 5**

| Reagent component | Concentration |
|---|---|
| MOPS buffer solution (pH7.5) | 1750 mmol/L |
| Glucose dehydrogenate | 2800 U/mL |
| Diaphorase | 1400 U/mL |
| Triton-X | 0.1 vol% |
| Distilled water | |

**Table 6**

| Formulation | Drying accelerator/Reagent stabilizing agent | Concentration |
|---|---|---|
| A | Not contained | |
| B | Sucrose | 10 vol% |
| C | Sucrose | 15 vol% |
| D | Sucrose | 20 vol% |
| E | Tetrasodium borate | 2 vol% |
| F | Tetrasodium borate /sucrose | 2 vol%/15 Vol% |

As understood from FIG. 16A, in the formulation A, wherein neither any drying accelerator nor any reagent stabilizing agent was added, no separation of the reagent in the reagent layers was recognized. However, as illustrated in FIG. 16B, it was confirmed that when the reagent layers were rubbed with the needle, the reagent layers contained a large amount of water and thus the reagent layers were hardly dried.

As understood from FIG. 17A, in the formulation B, wherein sucrose as a reagent stabilizing agent for making the drying period short was added to the reagent-containing liquid (except sucrose) in an amount of 10% by volume of the liquid, no separation of the reagent in the reagent layers was recognized. However, as illustrated in FIG. 17B, it was confirmed that when the reagent layers were rubbed with the needle, the reagent-containing liquid was not very much dried and thus the drying was hardly promoted.

As understood from FIG. 18A, in the formulation C, wherein sucrose as a reagent stabilizing agent was added to the reagent-containing liquid (except sucrose) in an amount of 15% by volume of the liquid, no separation of the reagent in the reagent layers was recognized. However, as illustrated in FIG. 18B, it was confirmed that when the reagent layers were rubbed with the needle, the reagent layers were soft and contained a large amount of water so that the reagent-containing liquid was not very much dried and the drying was not sufficiently promoted.

As understood from PIG. 19A, in the formulation D, wherein sucrose as a reagent stabilizing agent was added to the reagent-containing liquid (except sucrose) in an amount of 20% by volume of the liquid, irregularities made their appearance in the surfaces of the reagent layers. On the other hand, as illustrated in FIG. 19B, it was confirmed that when the reagent layers were rubbed with a needle, the drying of the reagent-containing liquid was promoted. In other words, when sucrose as a reagent stabilizing agent was added, the drying of the reagent-containing liquid was not promoted and further the surface property of the reagent layers deteriorated unless the addition amount thereof was made large.

On the other hand, as understood from FIG. 20A, in the formulation E, wherein tetrasodium borate as a drying accelerator was added to the reagent-containing liquid (except tetrasodium borate) in an amount of 2% by volume of the liquid, neither separation of the reagent in the reagent layers nor deterioration of the surface property (generation of irregularities or cracks) was recognize. As illustrated in FIG. FIG. 20B, when the reagent layers were rubbed with the needle, the reagent layers were not completely dried but the drying of the reagent-containing liquid was further promoted than the case where sucrose as a reagent stabilizing agent was added in an amount of 15% by volume. In other words, it was confirmed that the case where tetrasodium borate was used as a drying accelerator, as compared with the case where sucrose was used as a reagent stabilizing agent, drying promoting effect equal to or larger than that based on sucrose was obtained even if the addition amount was small, and further the surface property of the reagent layers did not deteriorate.

As illustrated in FIG. 21A, in the formulation F, wherein tetrasodium borate and sucrose as drying accelerators were added to the reagent-containing liquid (except tetrasodium borate and sucrose) in amounts of 2% by volume and 15% by volume of the liquid, respectively, the surface property of the reagent layers was made better than the case where sucrose alone was added. As illustrated in FIG. 21B, it was confirmed that the reagent layers were completely dried.

From the above-mentioned results, it has been understood that when tetrasodium borate is added as a drying accelerator, the drying of the reagent-containing layers is promoted and further the drying promotion effect is more effectively obtained on the basis of a smaller addition amount than when sucrose is added as a reagent stabilizing agent. Additionally, it has been understood that when tetrapotassium borate as a drying accelerator and sucrose as a reagent stabilizing agent are used together, the surface property is made better than when sucrose is used alone.

It is assumed that such drying promotion effect is obtained when tetrapotassium borate is used as a drying accelerator in the same manner as in Example 1. Moreover, when tetrasodium borate was added as a drying accelerator, separation of the reagent layers was not generated in the reagent layers and irregularities and cracks were not generated in the surfaces. Thus, it is assumed that in the same way as in Example 2, the generation of air bubbles is restrained when the reagent layers are dissolved, so that the measurement reproducibility is improved.

### Example 4

In the present example, drying promotion effect was examined in a case where tetrasodium borate was added when reagent layers suitable for measuring lactate dehydrogenate (LDH) were formed.

The reagent layers were formed by causing each reagent-containing liquid to adhere in a dot form onto a substrate made of polystyrene (PS), and then drying the resultant in a night. The reagent-containing liquid was prepared by gadding a drying accelerator shown in Table 8 described below to a basic composition shown in Table 7 described below. The dot-adhesion of the reagent-containing liquid was performed, using a dispenser. The dot-adhesion amount thereof was set to 50 nL.

The drying promotion effect was judged by checking the property of the reagent layers after the drying, and the property thereof after the reagent layers after the drying were rubbed with a needle. Optically microscopic photographs of the reagent layers after the drying are shown in FIG. 22A and FIG. 23A, and optically microscopic photographs of the reagent layers after the reagent layers were rubbed with the needle are shown in FIG. 22B and FIG. 23B.

**Table 7**

| Reagent component | Concentration |
|---|---|
| MEG | 2160 mmol/L |
| Lithium lactate | 200 mmol/L |
| CaCl₂·26H₂O | 5 wt% |
| Distilled water | |
| CHAPSO | 0.8 vol% |

| | |
|---|---|
| The pH was adjusted to 9.4 with 5 N HCl. MEG: N-methyl-D-glucamine CHAPSO: 3-[(3-cholamidopropyl)dimethylammonio]-2-hydroxypropanesulf onate | |

**Table 8**

| Formulation | Drying accelerator | Concentration |
|---|---|---|
| A | Not contained | |
| B | Tetrasodium borate | 5 vol% |

As understood from FIG. 22A, in the formulation A, wherein no drying accelerator was added, no separation of the reagent in the reagent layers was recognized. However, as illustrated in FIG. 22B, it was confirmed that when the reagent layers were rubbed with the needle, the reagent layers were not completely dried.

On the other hand, as understood from FIG. 23A, in the formulation B, wherein tetrasodium borate as a drying accelerator was added to the reagent-containing liquid (except tetrasodium borate) in an amount of 5% by volume of the liquid, no separation of the reagent in the reagent layers was recognized. Moreover, as illustrated in FIG. 23B, it was confirmed that when the reagent layers were rubbed with the needle, the drying of the reagent layers was further promoted as compared with the case where no drying accelerator was added, so that the reagent layers were completely dried in a night.

From the above-mentioned results, it has been understood that: in a case where reagent layers suitable for measuring lactate dehydrogenate (LDH) are formed on the basis of the above-mentioned basic composition in Table 7, also, the drying of the reagent-containing layers is promoted, without generating any separation of the reagent component in the reagent layers, irregularities in the surfaces nor cracks in the surfaces, when tetrasodium borate as a drying accelerator is added.

It is assumed that such drying promotion effect is obtained also when tetrapotassium borate is used as a drying accelerator in the same way as in Example 1 or when tetrasodium borate as a drying accelerator and sucrose as a reagent stabilizing agent are used together in the same way as in Example 3. Moreover, when tetrasodium borate was added as a drying accelerator, no separation of the reagent component was generated in the reagent layers and irregularities and cracks were not generated in the surfaces. Thus, it is assumed that in the same way as in Example 2, the generation of air bubbles is restrained when the reagent layers are dissolved, so that the measurement reproducibility is improved.

In Examples 1 to 4 described above, the drying promotion effect of tetrasodium borate and tetrapotassium borate were examined about the cases where the reagent layers the measurement targets of which were ALP, Glu and LDH were formed on the basis of the basic compositions shown in Tables 1, 5 and 7. However, the present invention can be applied not only to the cases where the reagent layers for the above-mentioned measurement targets are formed, or cases where the reagent layers for the above-mentioned measurement targets are formed on the basis of the basic compositions shown in Tables 1, 5 and 7 but also to cases where reagent layers suitable for measuring other components are formed, or cases where reagent layers for the above-mentioned measurement targets are formed from any composition other than the compositions in Tables 1 to 3.

## Claims

1. A method for forming a reagent layer in an analytical tool, comprising a first step of holding a reagent-containing liquid onto a substrate, and
a second step of drying the reagent-containing liquid,
wherein as the reagent-containing liquid, there is used a liquid containing a tetraborate as a drying accelerator.

2. The method for forming a reagent layer according to claim 1, wherein the content of the tetraborate in the reagent-containing liquid is 1 g or more based on 100 mL of the reagent-containing liquid.

3. The method for forming a reagent layer according to claim 2, wherein the content of the tetraborate in the reagent-containing liquid is from 1 to 10 g based on 100 mL of the reagent-containing liquid.

4. The method for forming a reagent layer according to claim 1, wherein the tetraborate is tetrasodium borate or tetrapotassium borate.

5. The method for forming a reagent layer according to claim 1, which further comprises sucrose.

6. The method for forming a reagent layer according to claim 5, wherein the content of sucrose in the reagent-containing liquid is from 5 to 15 g based on 100 mL of the reagent-containing liquid.

7. The method for forming a reagent layer according to claim 1, wherein the reagent-containing liquid contains at least one of an enzyme and a color-developing agent.

8. The method for forming a reagent layer according to claim 1, wherein the reagent-containing liquid is held in an amount of 10 to 100 nL on the substrate in the first step.

9. The method for forming a reagent layer according to claim 1, wherein the reagent-containing liquid is dried in an environment having a relative humidity of 10% or less in the second step.

10. The method for forming a reagent layer according to claim 9, wherein the reagent-containing liquid is dried at room temperature in the second step.

11. The method for forming a reagent layer according to claim 1, wherein the reagent layer is a layer for analyzing alkali phosphatase, glucose or lactate dehydrogenate.

12. The method for forming a reagent layer according to claim 1, wherein the reagent layer is formed as a single layer.

13. The method for forming a reagent layer according to claim 1, wherein the reagent layer is formed as a solid layer that is dissolved by a sample.

14. A method for producing an analytical tool having a reagent layer for being caused to react with a sample, comprising
a first step of holding a reagent-containing liquid on a substrate, and
a second step of drying the reagent-containing liquid,
wherein as the reagent-containing liquid, there is used a liquid containing a tetraborate as a drying accelerator.

15. The method for producing an analytical tool according to claim 14, wherein the analytical tool is a tool having a reaction tank for causing the sample and the reagent to react with each other, and
the reagent layer is provided in the reaction tank.

16. The method for producing an analytical tool according to claim 14, wherein the analytical tool is a tool used to analyze the sample in an optical technique.

17. An analytical tool having a reagent layer for causing a sample and a reagent to react with each other,
wherein the reagent layer contains a tetraborate.

18. The analytical tool according to claim 17, wherein a content of tetraborate in the reagent layer is from 3 to 15% by weight.

19. The analytical tool according to claim 17, wherein the tetraborate is tetrasodium borate or tetrapotassium borate.

20. The analytical tool according to claim 17, which further comprises sucrose.

21. The analytical tool according to claim 20, wherein a content of sucrose in the reagent layer is from 5 to 30% by weight.

22. The analytical tool according to claim 17, wherein the reagent layer is formed as a single layer.

23. The analytical tool according to claim 17, wherein the reagent layer is formed as a solid layer that is dissolved by the sample.

24. The analytical tool according to claim 17, which further comprises a reaction tank for causing the sample and the reagent to react with each other, and
the reagent layer is provided in the reaction tank.

25. The analytical tool according to claim 17, which is a tool for analyzing the sample in an optical technique.
